# EUROPEAN PATENT APPLICATION

(11) **EP 3 594 956 A1**
(43) Date of publication of application: **15.01.2020**
(21) Application number: 19182793.0
(22) Date of filing: 27.06.2019
(51) Int. Cl.: G16H 10/60, G06F 17/18

(54) **DEVICE AND METHOD TO DETERMINE BLOOD GLUCOSE SENSING DATA**

(30) Priority: 09.07.2018 KR 20180079111
(71) Applicant: Philosys Co., Ltd., Gunsan-si, Jeollabuk-do 54172 (KR)
(72) Inventor: PARK, Young Min, 06256 Seoul (KR); LEE, Seung Won, 13645 Gyeonggi-do (KR); LEE, Won Ju, 15071 Gyeonggi-do (KR)
(74) Representative: Stöckeler, Ferdinand

(57) **Abstract**

Provided is a device and method to determine blood glucose sensing data. The device may determine whether blood glucose sensing data is an error candidate based on a sampling value and a sampling slope of the blood glucose sensing data, and finally determine whether the blood glucose sensing data is error data by applying a blood glucose signal determination model to the error candidate.

## Description

### BACKGROUND

### 1. Field of the Invention

One or more example embodiments relate to technology for determining blood glucose sensing data.

### 2. Description of the Related Art

Recently, a growing number of modern people are suffering from so-called adult diseases such as diabetes, hyperlipemia, and thrombosis due to westernized dietary habits. Among young women, anemia patients with iron deficiency are increasing rapidly in number due to extreme diet. A simple method to know the severity of these adult diseases is measuring biocomponents in blood. The biocomponent measurement may detect quantities of a number of components in the blood, such as blood glucose, anemia, and blood coagulation, and thus an ordinary person may easily determine whether there is an abnormality, for example, whether a value of a specific component is in a normal region or an abnormal region, without seeing a doctor.

One easy biocomponent measurement is to quantitatively analyze an output signal using an electrochemical or photometric method after injecting a blood sample taken from a fingertip into a strip and inserting the strip into a biosensor. This biocomponent measurement is suitable for unprofessional ordinary people since a quantity of a corresponding component may be displayed on a measurement device.

The biosensor may also be used by being connected to a smart device. Thus, there is a demand for technology for accurately determining whether data sensed by the biosensor is an error.

### SUMMARY

An aspect provides a blood glucose sensing data determination device that may primarily classify blood glucose sensing data obtained from a biosensor as an error candidate.

An aspect provides a blood glucose sensing data determination device that may secondarily determine whether blood glucose sensing data classified as an error candidate is error data based on a blood glucose signal determination model.

An aspect provides a blood glucose sensing data determination device that may use a cloud server.

According to an aspect, there is provided a method of determining blood glucose sensing data, the method including collecting blood glucose sensing data corresponding to a predetermined time length, and determining whether the blood glucose sensing data is an error candidate, based on at least one of a sampling value and a sampling slope of the blood glucose sensing data.

The collecting may include receiving blood glucose sensing data including sampling values measured at a plurality of sampling points in a time series manner at predetermined time intervals.

The determining may include determining the blood glucose sensing data to be the error candidate, in response to an example in which a sampling value exceeding a threshold waiting value is detected during a waiting interval of the blood glucose sensing data.

The determining may include determining the blood glucose sensing data to be the error candidate, in response to an example in which a peak value of the blood glucose sensing data is out of a peak range.

The determining may include determining the blood glucose sensing data to be the error candidate, in response to an absence of a peak point during a peak interval of the blood glucose sensing data.

The determining may include determining the blood glucose sensing data to be the error candidate, in response to an example in which a sampling point having a negative sampling slope is detected before a peak point is detected.

The determining may include determining the blood glucose sensing data to be the error candidate, in response to an example in which a sampling value of a target sampling point is greater than or equal to a threshold reference value, and a sampling slope of the target sampling point is increased at a ratio exceeding a first threshold ratio when compared to a sampling slope of a previous sampling point during a diminishing interval.

The determining may include determining the blood glucose sensing data to be the error candidate, in response to an example in which a sampling value of a target sampling point is less than a threshold reference value, and a sampling slope of the target sampling point is increased to exceed a threshold absolute value during a diminishing interval.

The determining may include determining the blood glucose sensing data to be the error candidate, in response to an example in which a sampling point in the blood glucose sensing data is less than a threshold minimum value.

The determining may include determining the blood glucose sensing data to be the error candidate, in response to an example in which a sampling slope of a last sampling point in the blood glucose sensing data is increased at a ratio exceeding a second threshold ratio when compared to a sampling slope of a previous sampling point.

The determining may include determining the blood glucose sensing data to be the error candidate, in response to an example in which sampling points, of which a count is greater than or equal to a threshold succession count, having positive sampling slopes are successively detected during a diminishing interval of the blood glucose sensing data.

The determining may include determining the blood glucose sensing data to be the error candidate, in response to an example in which a count of sampling points having positive sampling slopes is greater than or equal to a threshold accumulation count during a diminishing interval of the blood glucose sensing data.

The method may further include determining whether the blood glucose sensing data is error data based on a blood glucose signal determination model, in response to an example in which the blood glucose sensing data is determined to be the error candidate.

The determining of whether the blood glucose sensing data is error data may include calculating a predicted blood glucose from the blood glucose sensing data based on the blood glucose signal determination model, detecting a measured blood glucose from the blood glucose sensing data, and determining whether the blood glucose sensing data is error data, based on a comparison between the predicted blood glucose and the measured blood glucose.

The determining of whether the blood glucose sensing data is error data may include calculating a difference between the predicted blood glucose and the measured blood glucose, and determining that the blood glucose sensing data is error data, in response to an example in which the calculated difference exceeds a threshold difference.

The determining of whether the blood glucose sensing data is error data may include loading, from a database, reference data of a range corresponding to a blood glucose value indicated by the blood glucose sensing data based on the blood glucose value, and verifying the blood glucose sensing data with respect to the loaded reference data, based on the blood glucose signal determination model.

The determining of whether the blood glucose sensing data is error data may include calculating results indicating whether the blood glucose sensing data is error data from at least one of a multi-layer perceptron (MLP) model, a dynamic time warping (DTW) model, a convolutional neural network (CNN), a recurrent neural network (RNN), a principal component analysis (PCA) model, a K-means clustering model, a support vector machine (SVM), and an isolation forest, and determining the blood glucose sensing data to be error data, in response to an example in which a majority of the calculated results indicates that the blood glucose sensing data is error data.

According to an aspect, there is provided a device for determining blood glucose sensing data, the device including a data collector configured to collect blood glucose sensing data corresponding to a predetermined time length, and a processor configured to determine whether the blood glucose sensing data is an error candidate, based on at least one of a sampling value and a sampling slope of the blood glucose sensing data.

Additional aspects of example embodiments will be set forth in part in the description which follows and, in part, will be apparent from the description, or may be learned by practice of the disclosure.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1 is a block diagram illustrating a configuration of a blood glucose sensing data determination system according to an example embodiment;
FIG. 2 is a flowchart illustrating a blood glucose sensing data determination method according to an example embodiment;
FIG. 3 illustrates blood glucose sensing data according to an example embodiment;
FIG. 4 is a flowchart illustrating an example of determining whether blood glucose sensing data is an error candidate according to an example embodiment;
FIGS. 5 through 9 illustrate examples of a blood glucose signal determination model used to determine whether blood glucose sensing data determined to be an error candidate is error data according to an example embodiment;
FIG. 10 illustrates varied waveforms of blood glucose sensing data according to an example embodiment; and
FIG. 11 illustrates an example of blood glucose sensing data verification using a blood glucose signal determination model according to an example embodiment.

### DETAILED DESCRIPTION

Hereinafter, some example embodiments will be described in detail with reference to the accompanying drawings. However, various alterations and modifications may be made to the example embodiments. Here, the example embodiments are not construed as limited to the disclosure and should be understood to include all changes, equivalents, and replacements within the idea and the technical scope of the disclosure.

The terminology used herein is for the purpose of describing particular examples only and is not to be limiting of the examples. As used herein, the singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which examples belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the examples with reference to the accompanying drawings, like reference numerals refer to like constituent elements and a repeated description related thereto will be omitted. In the description of example embodiments, detailed description of well-known related structures or functions will be omitted when it is deemed that such description will cause ambiguous interpretation of the present disclosure.

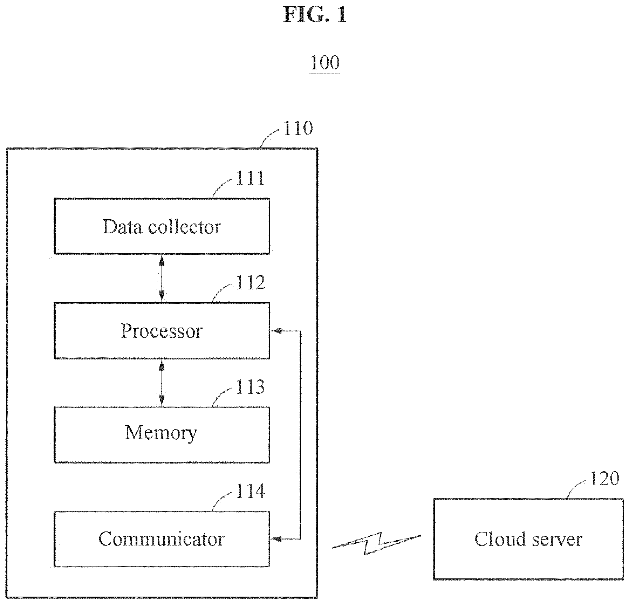

FIG. 1 is a block diagram illustrating a configuration of a blood glucose sensing data determination system according to an example embodiment.

A blood glucose sensing data determination system 100 may include a blood glucose sensing data determination device 110 and a cloud server 120.

The blood glucose sensing data determination device 110 may include a data collector 111, a processor 112, a memory 113, and a communicator 114. The blood glucose sensing data determination device 110 may be implemented as a mobile terminal. However, example embodiments are not limited thereto.

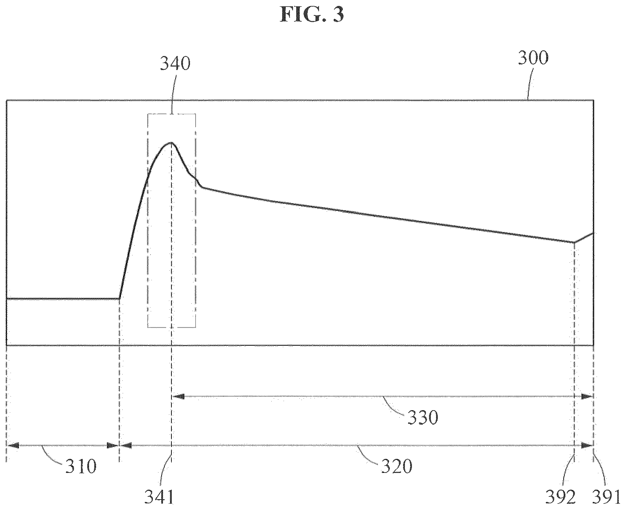

The data collector 111 may collect blood glucose sensing data corresponding to a predetermined time length. The data collector 111 may collect the blood glucose sensing data from a biosensor, for example, a test strip coated with a substance to detect a blood glucose. The data collector 111 may receive a blood glucose sensing signal which is an analog value from the biosensor, and obtain the blood glucose sensing data by converting the blood glucose sensing signal into a digital value. The blood glucose sensing data may be data in which the blood glucose sensing signal is quantized into a digital value. The time length of the blood glucose sensing data may vary depending on a design. The blood glucose sensing data will be described further below with reference to FIG. 3.

The biosensor may generate the blood glucose sensing signal indicating an amount of a target analyte. Herein, the analyte may be a material associated with a living body. The analyte may be a substance which is a subject of analysis. For example, the analyte is principally blood glucose herein. However, example embodiments are not limited thereto.

For example, the blood glucose sensing data determination device 110 may apply a working signal, for example, a working voltage, to the biosensor. The biosensor may output the blood glucose sensing signal in response to the application of the working signal. The blood glucose sensing signal may be, for example, in a form of current signal.

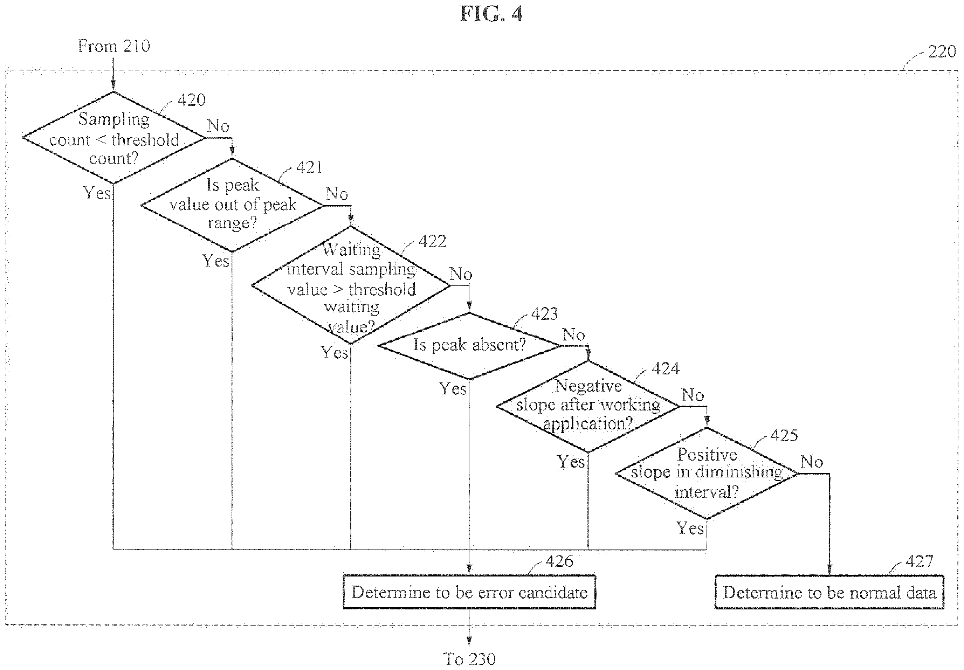

The processor 112 may determine whether the blood glucose sensing data is an error candidate based on at least one of a sampling value and a sampling slope of the blood glucose sensing data. The sampling value may be a value at a predetermined sampling point in the blood glucose sensing data. The sampling slope may be a slope at a predetermined sampling point in the blood glucose sensing data and may be, for example, a difference value between a value at a target sampling point and a value at a previous sampling point. The error candidate may be candidate data which preliminarily has a possibility of being error data among the blood glucose sensing data. An operation of determining whether the blood glucose sensing data is an error candidate will be described below with reference to FIG. 4.

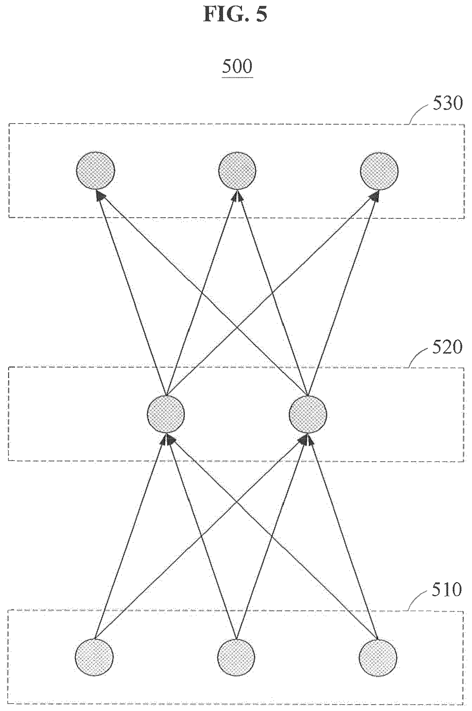

Further, the processor 112 may determine whether the blood glucose sensing data is error data based on a blood glucose signal determination model, in response to an example in which the blood glucose sensing data is determined to be an error candidate. Thus, the processor 112 may accurately determine whether the error candidate is error data using the blood glucose signal determination model. The blood glucose signal determination model will be described below with reference to FIGS. 5 through 9.

The memory 113 may temporarily or permanently store information needed to perform a blood glucose sensing data determination method. For example, the memory 113 may store the blood glucose signal determination model and the blood glucose sensing data.

The communicator 114 may communicate with the cloud server 120. For example, the communicator 114 may receive the blood glucose signal determination model and a parameter, for example, a trained connection weight, of the blood glucose signal determination model from the cloud server 120 through wired or wireless communication. Furthermore, the communicator 114 may update a measured blood glucose value to the cloud server 120.

The cloud server 120 may store a database associated with blood glucose. For example, the cloud server 120 may manage the blood glucose sensing data by dividing the blood glucose sensing data by blood glucose range. Further, the cloud server 120 may also store the blood glucose sensing data by dividing the blood glucose sensing data by user. The cloud server 120 may store the blood glucose signal determination model and the parameter of the blood glucose signal determination model. However, the description provided above is simply an example, and example embodiments are not limited thereto.

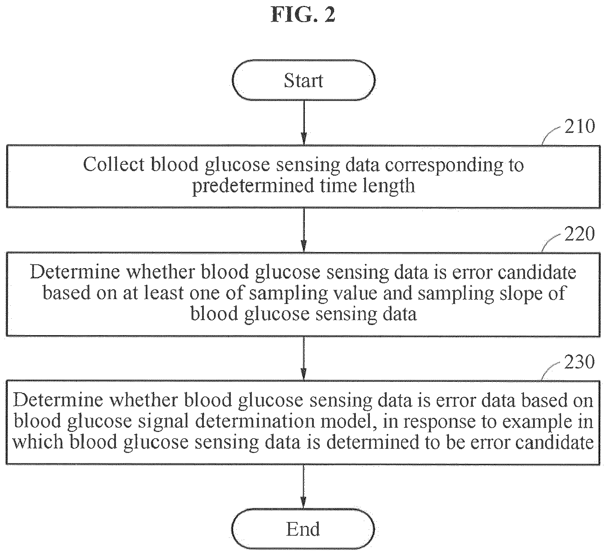

FIG. 2 is a flowchart illustrating a blood glucose sensing data determination method according to an example embodiment.

First, in operation 210, a blood glucose sensing data determination device may collect blood glucose sensing data corresponding to a predetermined time length. The blood glucose sensing data determination device may receive the blood glucose sensing data including sampling values measured at a plurality of sampling points in a time series at predetermined time intervals. For example, the blood glucose sensing data determination device may collect blood glucose sensing data corresponding to a predetermined time length of 5 seconds.

In operation 220, the blood glucose sensing data determination device may determine whether the blood glucose sensing data is an error candidate based on at least one of a sampling value and a sampling slope of the blood glucose sensing data. For example, the blood glucose sensing data determination device may compare the sampling value and the sampling slope of the blood glucose sensing data to a predetermined requirement, and determine the blood glucose sensing data to be an error candidate if the blood glucose sensing data satisfies the requirement. Error candidate determination will be described below with reference to FIG. 4.

In operation 230, the blood glucose sensing data determination device may determine whether the blood glucose sensing data is error data based on a blood glucose signal determination model, in response to an example in which the blood glucose sensing data is determined to be an error candidate. For example, the blood glucose sensing data determination device may calculate a final result indicating whether the blood glucose sensing data is error data using the blood glucose signal determination model with respect to the blood glucose sensing data determined to be an error candidate.

FIG. 3 illustrates blood glucose sensing data according to an example embodiment.

Blood glucose sensing data 300 may be data in which an analog value of a blood glucose sensing signal is converted into a digital value, as described above. The blood glucose sensing data 300 may have sampling values measured at a plurality of sampling points. Each sampling value may be a digital value, indicating a signal intensity of a biosensor, measured at an individual sampling point. A sampling point may be a point which separates a predetermined time length into equal intervals. For example, the predetermined time length of the blood glucose sensing data 300 may be 5 seconds, and the sampling points may be designated at intervals of 0.1 seconds. In this example, the blood glucose sensing data 300 may include 51 sampling points. A 51-st sampling point 391, which is the last sampling point, may have an average value for 5 seconds and 5.2 seconds as a sampling value. Thus, the 51-st sampling point 391 may have a sampling value which changes nonlinearly from a sampling value of a previous sampling point, for example, a 50-th sampling point 392. For example, the 51-st sampling point 391 may have a sampling value which is increased or decreased than the sampling value of the previous sampling point, for example, the 50-th sampling point 392.

In the blood glucose sensing data 300, a waiting interval 310 may be an interval between a point in time at which a measurement is started and a point in time at which a voltage is applied.

An applying interval 320 may be an interval after the voltage is applied. For example, when a voltage is applied to a biosensor, for example, a test strip, to which blood is applied, a current generated by the biosensor may sharply increase immediately after the voltage is applied. After a peak 341, the current generated by the biosensor may gradually decrease.

A peak interval 340 may be an interval in which the peak 341 is expected to appear after the voltage is applied. For example, when the blood glucose sensing data 300 includes 51 sampling points, the peak interval 340 may correspond to a 10-th sampling point to a 13-th sampling point.

A diminishing interval 330 may be an interval in which the sampling value tends to continuously decrease after the peak 341. However, as described above, the sampling value at the last sampling point may also be increased than the sampling value of the previous sampling point.

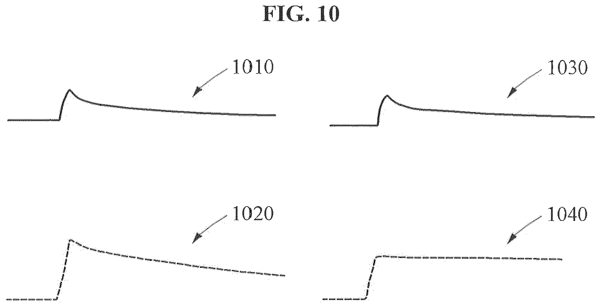

FIG. 3 illustrates a normal waveform of the blood glucose sensing data 300, and FIG. 10 describes varied waveforms.

FIG. 4 is a flowchart illustrating an example of determining whether blood glucose sensing data is an error candidate according to an example embodiment.

A blood glucose sensing data determination device may determine whether blood glucose sensing data is an error candidate based on a sampling value and a sampling slope of the blood glucose sensing data.

In operation 420, the blood glucose sensing data determination device may determine whether a sampling count is less than a threshold count. For example, in response to an example in which a count of sampling points included in the blood glucose sensing data is less than the threshold count, the blood glucose sensing data determination device may determine the blood glucose sensing data to be an error candidate, in operation 426. For example, if a predetermined time length is 5 seconds, and a time interval between sampling points is 0.1 seconds, the threshold count may be "51". If the total count of the sampling points in the blood glucose sensing data is less than the threshold count, it may indicate that a measurement with respect to at least one sampling point is omitted, and thus the blood glucose sensing data determination device may determine the blood glucose sensing data to be an error candidate.

In operation 421, the blood glucose sensing data determination device may determine whether a peak value of the blood glucose sensing data is out of a peak range. In response to an example in which the peak value of the blood glucose sensing data is out of the peak range, the blood glucose sensing data determination device may determine the blood glucose sensing data to be an error candidate, in operation 426. For example, the peak range may be a range in which 3 sigma is applied to a statistical average of peak values. The following Table 1 shows a distribution of peak values based on a sampling value at a last sampling point of the blood glucose sensing data. In Table 1, 3 sigma may indicate a range in which data of about 99.7% is allowed. 3CV may be three times a relative standard deviation. The relative standard deviation is a coefficient of variation (CV), which is a value obtained by dividing a standard deviation by an arithmetical mean. Although not shown in Table 1, an average peak value calculated with respect to a corresponding sampling value may be mapped to each last sampling value. For example, the peak range may be a range in which 3 sigma is applied, based on the average peak value mapped to each last sampling value.

**[Table 1]**

| Last sampling value | 3 CV [%] | Last sampling value | 3 CV [%] | Last sampling value | 3 CV [%] |
|---|---|---|---|---|---|
| 234 | 42 | 1512 | 36 | 2790 | 21 |
| 376 | 31 | 1654 | 33 | 2932 | 20 |
| 518 | 35 | 1796 | 39 | 3074 | 22 |
| 660 | 46 | 1938 | 26 | 3216 | 21 |
| 802 | 49 | 2080 | 29 | 3358 | 18 |
| 944 | 50 | 2222 | 26 | 3500 | 17 |
| 1086 | 44 | 2364 | 25 | 3642 | 19 |
| 1228 | 43 | 2506 | 24 | 3784 | 19 |
| 1370 | 41 | 2648 | 20 | 3926 | 19 |

In Table 1, the blood glucose sensing data determination device may determine a 3CV corresponding to a value between last sampling values through a linear interpolation. With respect to a sampling value out of the provided sampling value range, a 3CV value may be fixed to a 3CV value at an end point. For example, with respect to blood glucose sensing data with a last sampling value less than or equal to "234", the blood glucose sensing data determination device may fix the 3CV value to 42%. With respect to blood glucose sensing data with a last sampling value greater than or equal to "3926", the blood glucose sensing data determination device may fix the 3CV value to 19%. However, Table 1 is simply an example, and example embodiments are not limited thereto.

In operation 422, the blood glucose sensing data determination device may determine whether a waiting interval count exceeds a threshold waiting count. For example, in response to an example in which a sampling value exceeding a threshold waiting value is detected during a waiting interval of the blood glucose sensing data, the blood glucose sensing data determination device may determine the blood glucose sensing data to be an error candidate, in operation 426. For example, the threshold waiting value may be "50". However, example embodiments are not limited thereto, and the threshold waiting value may vary depending on a design. Since a voltage is not applied yet in the waiting interval, the blood glucose sensing data determination device may determine the blood glucose sensing data to be an error candidate when a sampling value of an excessive size is detected.

In operation 423, the blood glucose sensing data determination device may determine whether a peak is absent. For example, in response to an absence of a peak point during a peak interval of the blood glucose sensing data, the blood glucose sensing data determination device may determine the blood glucose sensing data to be an error candidate, in operation 426. When the blood glucose sensing data includes 51 sampling points, the peak interval may correspond to, for example, 10-th to 13-th sampling points. Blood glucose sensing data including no peak may be potentially an error candidate.

In operation 424, the blood glucose sensing data determination device may determine whether a negative slope appears after working application. For example, in response to an example in which a sampling point having a negative sampling slope is detected before a peak point is detected, the blood glucose sensing data determination device may determine the blood glucose sensing data to be an error candidate, in operation 426. The negative sampling slope may be, for example, a slope less than "0". A current should sharply increase from the 10-th sampling point to the peak point immediately after a working voltage is applied. Thus, blood glucose sensing data in which a negative slope appears before a peak may be an error candidate.

In operation 425, the blood glucose sensing data determination device may determine whether a positive slope appears in a diminishing interval. For example, the blood glucose sensing data determination device may determine whether an abnormal slope exists during an interval from a point in time at which the working voltage is applied to 5 seconds.

For example, in response to an example in which a sampling value of a target sampling point is greater than or equal to a threshold reference value, and a sampling slope of the target sampling point is increased at a ratio exceeding a first threshold ratio when compared to a sampling slope of a previous sampling point during a diminishing interval, the blood glucose sensing data determination device may determine the blood glucose sensing data to be an error candidate, in operation 426. The target sampling point may be a sampling point which is sampled within the diminishing interval. The threshold reference value may be, for example, "500", and the first threshold ratio may be, for example, 5%. A sampling point having a sampling value greater than the threshold reference value may be less affected by noise, and thus the blood glucose sensing data determination device may determine whether the blood glucose sensing data is an error candidate based on a slope change rate between the target sampling point and the previous sampling point.

For example, in response to an example in which a sampling value of a target sampling point is less than a threshold reference value, and a sampling slope of the target sampling point is increased to exceed a threshold absolute value during the diminishing interval, the blood glucose sensing data determination device may determine the blood glucose sensing data to be an error candidate, in operation 426. The threshold absolute value may be, for example, "25". However, example embodiments are not limited thereto, and the threshold absolute value may vary depending on a design. A sampling point having a sampling value less than the threshold reference value may be relatively more affected by noise, and thus the blood glucose sensing data determination device may determine whether the blood glucose sensing data is an error candidate based on a difference in absolute values between the target sampling point and a previous sampling point.

For example, in response to an example in which a sampling point in the blood glucose sensing data is less than a threshold minimum value, the blood glucose sensing data determination device may determine the blood glucose sensing data to be an error candidate, in operation 426. The threshold minimum value may be, for example, "82". However, example embodiments are not limited thereto. The threshold minimum value may be a value corresponding to a blood injection threshold of a blood glucose measurer. When the sampling value is diminutive, the blood glucose sensing data may be an error candidate.

For example, in response to an example in which a sampling slope of a last sampling point in the blood glucose sensing data is increased at a ratio exceeding a second threshold when compared to a sampling slope of a previous sampling point, the blood glucose sensing data determination device may determine the blood glucose sensing data to be an error candidate, in operation 426. The second threshold ratio may be, for example, 10%. However, example embodiments are not limited thereto. The second threshold ratio may be set to be greater than the first threshold ratio described above. That is because the last sampling point may be, for example, a mean value between 5 seconds and 5.2 seconds, and thus have a relatively great value when compared to the previous sampling point. The sampling value of the last sampling point may be a value obtained by compensating for a parasitic resistance component of an operational amplifier (OP amplifier).

For example, in response to an example in which sampling points, of which a count is greater than or equal to a threshold succession count, having positive sampling slopes are successively detected during a diminishing interval of the blood glucose sensing data, the blood glucose sensing data determination device may determine the blood glucose sensing data to be an error candidate, in operation 426. The positive sampling slope may be a slope greater than "0". For example, the threshold succession count may be "4". Blood glucose sensing data having a sampling value which constantly increases at a slope less than the first threshold ratio may be an error candidate.

For example, in response to an example in which a count of sampling points having positive sampling slopes is greater than or equal to a threshold accumulation count during a diminishing interval of the blood glucose sensing data, the blood glucose sensing data determination device may determine the blood glucose sensing data to be an error candidate, in operation 426. The threshold accumulation count may be, for example, "10". If an excessive ripple waveform occurs due to noise or other factors, corresponding blood glucose sensing data may be an error candidate.

In operation 427, the blood glucose sensing data determination device may determine blood glucose sensing data not satisfying all of the requirements of operations 421 through 425 to be normal data. The blood glucose sensing data determination device may identify a blood glucose value of a user from the blood glucose sensing data determined to be normal data.

Although FIG. 4 sequentially illustrates the requirements with respect to the sampling value and the sampling slope, an order of the operations is not limited thereto. For example, the order of the operations of FIG. 4 may be changed, or partially omitted.

FIGS. 5 through 9 illustrate blood glucose signal determination models used to determine whether blood glucose sensing data determined to be an error candidate is error data according to an example embodiment.

A blood glucose sensing data determination device may finally determine whether blood glucose sensing data being an error candidate is error data using a blood glucose signal determination model, as described in operation 230 of FIG. 2. The blood glucose sensing data determination device may normalize the blood glucose sensing data determined to be an error candidate. For example, the blood glucose sensing data determination device may normalize the blood glucose sensing data to have a value between "0" and "1 ".

For example, the blood glucose sensing data determination device may calculate a predicted blood glucose from the blood glucose sensing data based on the blood glucose signal determination model. The blood glucose sensing data determination device may detect a measured blood glucose from the blood glucose sensing data. The blood glucose sensing data determination device may determine whether the blood glucose sensing data is error data based on a comparison between the predicted blood glucose and the measured blood glucose. In this example, the blood glucose signal determination model may be a model trained to output a predicted blood glucose from input blood glucose sensing data. The blood glucose sensing data determination device may calculate a difference between the predicted blood glucose and the measured blood glucose. In response to an example in which the calculated difference exceeds a threshold difference, the blood glucose sensing data determination device may determine that the blood glucose sensing data is error data.

For example, the blood glucose sensing data determination device may determine whether the blood glucose sensing data is error data from the blood glucose sensing data based on the blood glucose signal determination model. In this example, the blood glucose signal determination model may be a model trained to output whether the input blood glucose sensing data is normal or abnormal.

The blood glucose sensing data determination device may calculate results indicating whether the blood glucose sensing data is error data from at least one of a multi-layer perceptron (MLP) model, a dynamic time warping (DTW) model, a convolutional neural network (CNN), a recurrent neural network (RNN), a principal component analysis (PCA) model, a K-means clustering model, a support vector machine (SVM), and an isolation forest. The blood glucose sensing data determination device may determine the blood glucose sensing data to be error data, in response to an example in which a majority of the calculated results indicates that the blood glucose sensing data is error data. However, example embodiments are not limited thereto. The blood glucose sensing data determination device may determine whether the blood glucose sensing data is error data through an ensemble of the results output from the plurality of models.

In response to an example in which the blood glucose sensing data is determined to be normal data, the blood glucose sensing data determination device may determine a sampling value of a last sampling point in the blood glucose sensing data to be a final blood glucose value.

Hereinafter, individual examples of the blood glucose signal determination model will be described.

FIG. 5 illustrates an MLP model. For example, the MLP model may be a neural network designed to infer a type of new data by learning a pattern for each type of input data. The MLP model may allow a nonlinear function to pass through a hidden layer, thereby solving high-dimensional problems.

A brief structure of a neural network 500 will be described below.

The neural network 500 may include a plurality of layers including a plurality of nodes. Further, the neural network 500 may include connection weights to connect a plurality of nodes included in each of the plurality of layers to nodes included in another layer. A training device may obtain the neural network 500 from an internal database stored in a memory, or received from an external server through a communicator. The training device may be a device implemented independently of the blood glucose sensing data determination device. However, example embodiments are not limited thereto. The training device may be integrated into the blood glucose sensing data determination device.

For example, the neural network 500 may be a recognition model which simulates a calculation ability of a biological system using enormous artificial neurons connected by edges. The neural network 500 may be implemented as software, hardware, or a combination thereof. The neural network 500 may be referred to as an artificial neural network.

The neural network 500 may use artificial neurons that simplify functions of biological neurons. The artificial neurons may be referred to as nodes. The artificial neurons may be connected to each other through edges having connection weights. The connection weights may be predetermined values of the edges, and may be referred to as synapse weights or connection strengths.

The neural network 500 may include a plurality of layers. For example, the neural network 500 may include an input layer 510, a hidden layer 520, and an output layer 530. The input layer 510 may receive an input to perform training or recognition and transfer the received input to the hidden layer 520, and the output layer 530 may generate an output of the neural network 500 based on a signal received from the hidden layer 520. The hidden layer 520 may be disposed between the input layer 510 and the output layer 530, and change a training input of training data received from the input layer 510 into an easily predicable value.

Herein, training data may be a data set including a plurality of training pairs. For example, a training pair may include a training input and a training output, and the training output may be a value which should be output from the training input. Thus, the training data may include a plurality of training inputs and training outputs respectively mapped to the plurality of training inputs.

The input layer 510, the hidden layer 520, and the output layer 530 may each include a plurality of nodes. The nodes included in the input layer 510 may be referred to as input nodes, the nodes included in the hidden layer 520 may be referred to as hidden nodes, and the nodes included in the output layer 530 may be referred to as output nodes.

The input nodes included in the input layer 510 and the hidden nodes included in the hidden layer 520 may be connected to each other through edges having connection weights. The hidden nodes included in the hidden layer 520 and the output nodes included in the output layer 530 may be connected to each other through edges having connection weights.

Although not shown in the drawings, a neural network may include a plurality of hidden layers. A neural network including a plurality of hidden layers may be referred to as a deep neural network. Training the deep neural network may be referred to as deep learning. Assuming that the hidden layer 520 includes a first hidden layer, a second hidden layer, and a third hidden layer, an output of a hidden node belonging to the first hidden layer may be connected to hidden nodes belonging to the second hidden layer. An output of a hidden node belonging to the second hidden layer may be connected to hidden nodes belonging to the third hidden layer.

For example, the training device may input outputs of previous hidden nodes included in a previous hidden layer into each hidden layer through edges having connection weights, and generate outputs of hidden nodes included in the corresponding hidden layer based on values obtained by applying the connection weights to the outputs of the previous hidden nodes and activation functions. To fire an output to a next hidden node, a result of the activation functions needs to exceed a threshold of a current hidden node. In this example, a node may maintain a deactivated state without firing a signal to a next node until a predetermined threshold strength of activation is reached through input vectors.

The training device may train at least a portion of the neural network 500 through supervised learning. The training device may be implemented as software modules, hardware modules, or a combination thereof. Supervised learning may refer to a method of inputting a training input of training data and a corresponding training output into the neural network 500, and updating connection weights of edges so that output data corresponding to the training output of the training data may be output.

Further, the training device may train at least a portion of the neural network 500 through unsupervised learning. Unsupervised learning may refer to a method of calculating a loss, for example, an entropy loss, based on an output obtained by forward propagating a training input of training data, and updating connection weights of edges so that the loss may decrease.

Although FIG. 5 illustrates the neural network provided in a node structure, example embodiments are not limited to such a node structure. Various data structures may be used to store the neural network in a memory storage.

The training device may determine parameters of nodes included in the neural network through gradient descent based on output values of the nodes and a loss back-propagated to the neural network. For example, the training device may update the connection weights between the nodes through loss back-propagation learning. Loss back-propagation learning refers to a method of estimating a loss with respect to provided training data through forward computation, and updating connection weights to reduce the loss while propagating the estimated loss in a backward direction from the output layer 530 toward the hidden layer 520 and the input layer 510. Processing of the neural network 500 may be performed in an order of the input layer 510, the hidden layer 520, and the output layer 530. However, in the loss back-propagation learning, the connection weights may be updated in an order of the output layer 530, the hidden layer 520, and the input layer 510. To process the neural network as desired, one or more processors may use a buffer memory configured to store layers or a series of computed data.

The training device may define an objective function to be used to measure optimalities of currently set connection weights, continuously change the connection weights based on a result of the objective function, and iteratively perform training. For example, the objective function may be a loss function to be used by the neural network 500 to calculate a loss between an actual output value and a value expected to be output with respect to a training input of training data. The training device may update the connection weights to reduce a value of the loss function.

For example, a cloud server may have reference blood glucose data classified into a plurality of groups based on blood glucose concentrations. The reference blood glucose data may be classified based on the following blood glucose concentrations, for example, 50 milligrams per deciliter (mg/dL), 100 mg/dL, 150 mg/dL, 200 mg/dL, 300 mg/dL, 400 mg/dL, and 500 mg/dL. The reference blood glucose data may be labeled with reference concentrations corresponding to the respective groups. By setting the labeled data as a ground truth, the training device may train the neural network 500 with the reference blood glucose data. The training device may calculate an accuracy by calculating a matching degree of the ground truth and predicted data for performance evaluation. The training device may store the parameters used for training, for example, connection weights, and bias as parameter data. The parameter data may be stored in the cloud server and the blood glucose sensing data determination device. For example, the cloud server may receive the parameter data from the training device, and transfer the parameter data to the blood glucose sensing data determination device.

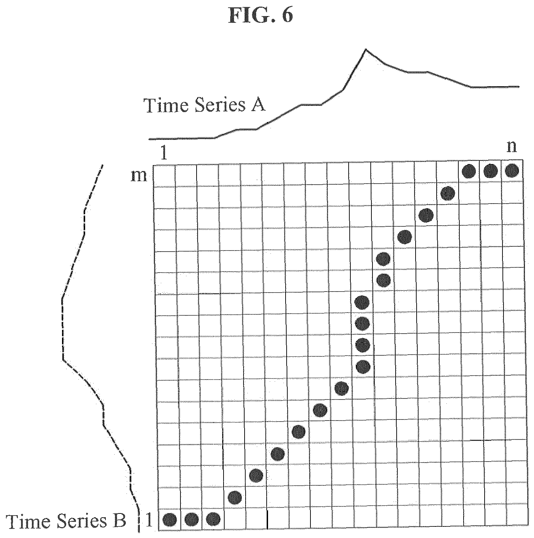

FIG. 6 illustrates a DTW model. The DTW model may be a model which analyzes a similarity of time series patterns. Referring to FIG. 6, a reference training device may fix reference data, and then generate an n x n matrix with time series data to be compared. In a state in which the matrix is filled with values by a predetermined conditional expression, two items of data with a length of path and a value of last (m, n) being close to "0" may be similar to each other. Here, m and n may be integers greater than or equal to "0".

The DTW model may be implemented as a K-nearest neighbor (K-NN) classifier. 1-NN may correspond to an example in which K of K-NN is "1". The K-NN classifier may be a non-parametric machine learning scheme. In a feature space, data may be an object assigned to an item most common among k nearest neighbors, the object classified by a majority vote. The K-NN classifier is based on a Euclidean distance, and thus may be used in low dimensions.

The blood glucose sensing data determination device may obtain a similarity to a reference time series using the DTW model, utilize the similarity as a metric, and classify values with a high similarity using K-NN with K=1 after obtaining all values of a single dimension.

For example, the blood glucose sensing data determination device may set a reference normal waveform which may represent each class. The blood glucose sensing data determination device may calculate a similarity between a waveform collected from a could server and the reference waveform using the DTW model. The blood glucose sensing data determination device may generate a data table of [similarity, blood glucose value]. The blood glucose sensing data determination device may train the 1-NN classifier with the data table. The blood glucose sensing data determination device may calculate an accuracy of the 1-NN classifier using experiment data not used for training. The blood glucose sensing data determination device may calculate a predicted blood glucose from new blood glucose sensing data based on the 1-NN classifier.

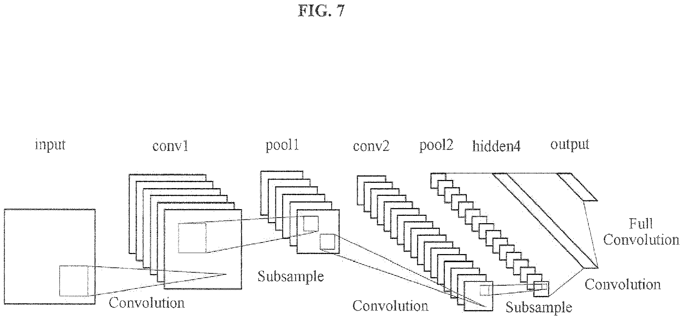

FIG. 7 illustrates a CNN.

A CNN may be principally used for image recognition and may also be applied to sentence recognition and signal processing. The CNN may apply a connection weight to each element only in a receptive field. The blood glucose sensing data determination device may reduce a dimension by compressing as much information as the size of the receptive field into a matrix of a desired size. Thus, the blood glucose sensing data determination device may reduce a dimension through convolutional layers, thereby reducing a width and a height of feature data. With the reduction in dimension, a depth corresponding to a number of kernels may increase.

The blood glucose sensing data determination device may set a kernel size to [1x10], and extract abstract values while reducing waveform features of the blood glucose sensing data. For example, the CNN may be trained only with information extracted from a fully connected layer which is a last output layer. The blood glucose sensing data determination device may calculate a predicted blood glucose from the blood glucose sensing data based on the CNN.

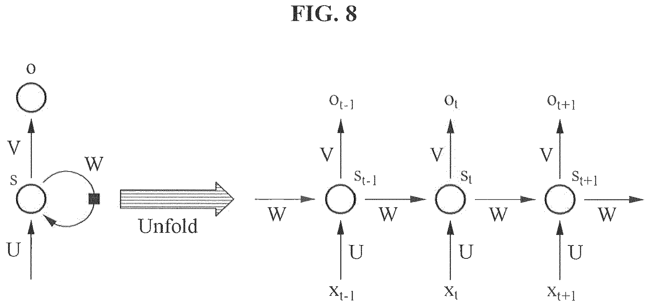

FIG. 8 illustrates an RNN.

An RNN may be principally applied to time series data recognition. The RNN may be trained by concurrently receiving a value of a point in time t-1, a value of a point in time t, and a value of a point in time t+1 in a predetermined single cell. The RNN may be trained to output a reference blood glucose value from reference blood glucose data. The blood glucose sensing data determination device may calculate a predicted blood glucose from the blood glucose sensing data based on the RNN. Here, t may be an integer greater than or equal to "2".

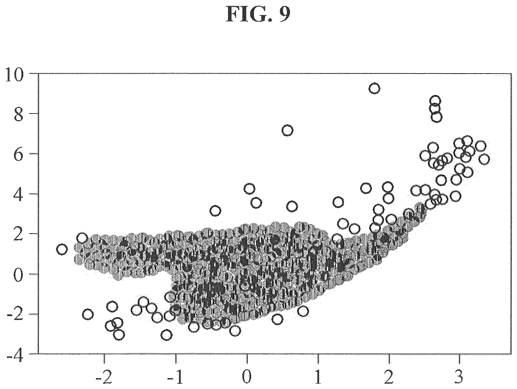

FIG. 9 illustrates a PCA model and a K-means clustering model.

A PCA model may be a model which reduces high-dimensional data to low-dimensional data. When the PCA model is used, a distribution of data may be visually represented on a two-dimensional coordinate plane. A K-means clustering model may be a model trained until an optimal center point is found after K center points designated by a user are set at random on the coordinate plane. Thereafter, a label corresponding to the center point may be mapped to data used for training, and thus data may be clustered by label. Here, K may be an integer greater than or equal to "1".

The blood glucose sensing data determination device may collect N values similar to a blood glucose value of the blood glucose sensing data from a cloud server. Here, N may be an integer greater than or equal to "1". After the PCA model is applied to the N values, the blood glucose sensing data determination device may apply the N values to the K-means clustering model, for example, a model with K=2, thereby verifying a group to which an individual value belongs. For example, a value positioned in a group to which a majority of data belongs may be normal data. In a converse case, the corresponding value may be error data.

FIG. 10 illustrates varied waveforms of blood glucose sensing data according to an example embodiment.

For example, first blood glucose sensing data 1010 and third blood glucose sensing data 1030 may have low blood glucose values. In the first blood glucose sensing data 1010 and the third blood glucose sensing data 1030, a sampling value may sharply decrease after a peak point, and noise may occur in a diminishing interval.

Second blood glucose sensing data 1020 may represent a high blood glucose value. In the second blood glucose sensing data 1020, a sampling value may gently decrease after a peak point.

Fourth blood glucose sensing data 1040 may represent error data. The fourth blood glucose sensing data 1040 may include neither peak point nor a diminishing interval.

The blood glucose sensing data determination device may primarily determine whether blood glucose sensing data showing a different waveform depending on a blood glucose value as shown in FIG. 10 is an error candidate, and secondarily and finally determine whether the blood glucose sensing data is error data. Thus, the blood glucose sensing data determination device may use a blood glucose value of blood glucose sensing data determined to be an error candidate if the blood glucose sensing data is determined to be normal data.

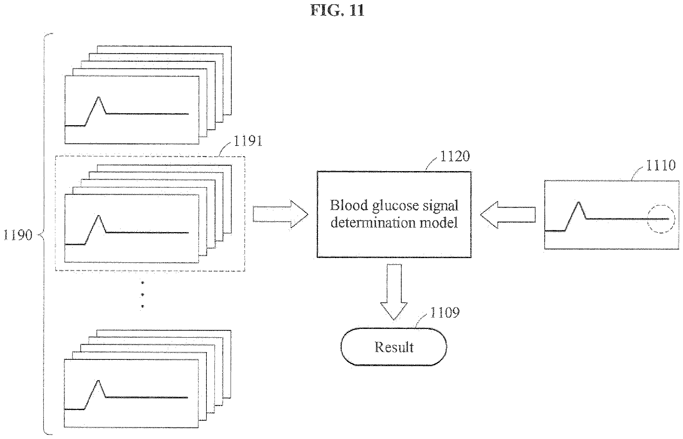

FIG. 11 illustrates an example of blood glucose sensing data verification using a blood glucose signal determination model according to an example embodiment.

A blood glucose sensing data determination device may load, from a database 1190, reference data 1191 of a range corresponding to a blood glucose value indicated by blood glucose sensing data 1110 based on the blood glucose value. The range corresponding to the blood glucose value may be a range including the corresponding blood glucose value. For example, the blood glucose sensing data determination device may identify the blood glucose value from a last sampling point of the blood glucose sensing data 1110. A cloud server may include the database 1190 containing the reference data 1191 individually labeled with a reference blood glucose value. The blood glucose sensing data determination device may request the reference data 1191 of the range corresponding to the blood glucose value from the cloud server. For example, when a measured blood glucose value is 70 mg/dL, the blood glucose sensing data determination device may receive, from the cloud server, reference data 1191 corresponding to a range of 60 mg/dL to 80mg/dL.

The blood glucose sensing data determination device may verify the blood glucose sensing data 1110 with respect to the loaded reference data 1191 based on a blood glucose signal determination model 1120. For example, the blood glucose sensing data determination device may calculate a result 1109, for example, a similarity, indicating whether the blood glucose sensing data 1110 is similar to the reference data 1191 based on the blood glucose signal determination model 1120. The blood glucose sensing data determination device may determine the corresponding blood glucose sensing data 1110 to be normal data when the blood glucose sensing data 1110 being an error candidate is determined to be similar to the reference data 1191 within the range. The blood glucose sensing data determination device may determine the corresponding blood glucose sensing data 1110 to be error data when the blood glucose sensing data 1110 is determined to be dissimilar to the reference data 1191.

Thus, the blood glucose sensing data determination device may use reference data 1191 having a blood glucose value of a range adjacent to a currently measured blood glucose sensing data 1110, in view of a characteristic of a waveform which varies depending on a blood glucose value. The blood glucose sensing data determination device may more accurately determine whether the blood glucose sensing data 1110 is error data.

According to example embodiments, a blood glucose sensing data determination device may fast determine whether blood glucose sensing data obtained from a biosensor is an error candidate based on a sampling value and a sampling slope of the blood glucose sensing data.

According to example embodiments, a blood glucose sensing data determination device may accurately determine whether an error candidate is error data by utilizing various blood glucose signal determination models.

According to example embodiments, a blood glucose sensing data determination device may save a memory by receiving parameter data and reference data through a cloud server only if needed.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher level code that may be executed by the computer using an interpreter. The above-described devices may be configured to act as one or more software modules in order to perform the operations of the above-described example embodiments, or vice versa.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A method of determining blood glucose sensing data, the method comprising:
collecting blood glucose sensing data corresponding to a predetermined time length; and
determining whether the blood glucose sensing data is an error candidate, based on at least one of a sampling value and a sampling slope of the blood glucose sensing data.

2. The method of claim 1, wherein the collecting comprises receiving blood glucose sensing data including sampling values measured at a plurality of sampling points in a time series manner at predetermined time intervals.

3. The method of claim 1, wherein the determining comprises determining the blood glucose sensing data to be the error candidate, in response to an example in which a sampling value exceeding a threshold waiting value is detected during a waiting interval of the blood glucose sensing data.

4. The method of claim 1, wherein the determining comprises determining the blood glucose sensing data to be the error candidate, in response to an example in which a peak value of the blood glucose sensing data is out of a peak range.

5. The method of claim 1, wherein the determining comprises comprises determining the blood glucose sensing data to be the error candidate, in response to an absence of a peak point during a peak interval of the blood glucose sensing data.

6. The method of claim 1, wherein the determining comprises determining the blood glucose sensing data to be the error candidate, in response to an example in which a sampling point having a negative sampling slope is detected before a peak point is detected.

7. The method of claim 1, wherein the determining comprises determining the blood glucose sensing data to be the error candidate, in response to an example in which a sampling value of a target sampling point is greater than or equal to a threshold reference value, and a sampling slope of the target sampling point is increased at a ratio exceeding a first threshold ratio when compared to a sampling slope of a previous sampling point during a diminishing interval.

8. The method of claim 1, wherein the determining comprises determining the blood glucose sensing data to be the error candidate, in response to an example in which a sampling value of a target sampling point is less than a threshold reference value, and a sampling slope of the target sampling point is increased to exceed a threshold absolute value during a diminishing interval.

9. The method of claim 1, wherein the determining comprises determining the blood glucose sensing data to be the error candidate, in response to an example in which a sampling point in the blood glucose sensing data is less than a threshold minimum value.

10. The method of claim 1, wherein the determining comprises determining the blood glucose sensing data to be the error candidate, in response to an example in which a sampling slope of a last sampling point in the blood glucose sensing data is increased at a ratio exceeding a second threshold ratio when compared to a sampling slope of a previous sampling point.

11. The method of claim 1, wherein the determining comprises determining the blood glucose sensing data to be the error candidate, in response to an example in which sampling points, of which a count is greater than or equal to a threshold succession count, having positive sampling slopes are successively detected during a diminishing interval of the blood glucose sensing data.

12. The method of claim 1, wherein the determining comprises determining the blood glucose sensing data to be the error candidate, in response to an example in which a count of sampling points having positive sampling slopes is greater than or equal to a threshold accumulation count during a diminishing interval of the blood glucose sensing data.

13. The method of claim 1, further comprising:
determining whether the blood glucose sensing data is error data based on a blood glucose signal determination model, in response to an example in which the blood glucose sensing data is determined to be the error candidate.

14. The method of claim 13, wherein the determining of whether the blood glucose sensing data is error data comprises:
calculating a predicted blood glucose from the blood glucose sensing data based on the blood glucose signal determination model;
detecting a measured blood glucose from the blood glucose sensing data; and
determining whether the blood glucose sensing data is error data, based on a comparison between the predicted blood glucose and the measured blood glucose.

15. The method of claim 14, wherein the determining of whether the blood glucose sensing data is error data comprises:
calculating a difference between the predicted blood glucose and the measured blood glucose; and
determining that the blood glucose sensing data is error data, in response to an example in which the calculated difference exceeds a threshold difference.

16. The method of claim 13, wherein the determining of whether the blood glucose sensing data is error data comprises:
loading, from a database, reference data of a range corresponding to a blood glucose value indicated by the blood glucose sensing data based on the blood glucose value; and
verifying the blood glucose sensing data with respect to the loaded reference data, based on the blood glucose signal determination model.

17. The method of claim 13, wherein the determining of whether the blood glucose sensing data is error data comprises:
calculating results indicating whether the blood glucose sensing data is error data from at least one of a multi-layer perceptron (MLP) model, a dynamic time warping (DTW) model, a convolutional neural network (CNN), a recurrent neural network (RNN), a principal component analysis (PCA) model, a K-means clustering model, a support vector machine (SVM), and an isolation forest; and
determining the blood glucose sensing data to be error data, in response to an example in which a majority of the calculated results indicates that the blood glucose sensing data is error data.

18. A device for determining blood glucose sensing data, the device comprising:
a data collector configured to collect blood glucose sensing data corresponding to a predetermined time length; and
a processor configured to determine whether the blood glucose sensing data is an error candidate, based on at least one of a sampling value and a sampling slope of the blood glucose sensing data.
